# EUROPÄISCHE PATENTSCHRIFT

(11) **EP 1 508 289 B1**
(45) Veröffentlichungstag und Bekanntmachung des Hinweises auf die Patenterteilung: **13.12.2006**
(21) Anmeldenummer: 04018593.6
(22) Anmeldetag: 05.08.2004
(51) Int. Cl.: A47F 3/04, A61L 9/015

(54) **Gekühltes Warenpräsentationsmöbel mit einem Luftaufbereitungssystem und Verfahren zum Betreiben eines derartigen Warenpräsentationsmöbels**
Refrigerated show case with an air treatment system and method to operate such a show case
Meuble de présentation réfrigéré avec un système pour le traitement d'air et méthode d'opération d'un tel meuble de présentation

(30) Priorität: 19.08.2003 DE 10338096
(43) Veröffentlichungstag der Anmeldung: 23.02.2005
(73) Patentinhaber: Linde Kältetechnik GmbH & Co.KG, 50999 Köln (DE)
(72) Erfinder: Klippel, Gabriele, 55288 Partenheim (DE); Alt, Carl Heinz, 55246 Mainz-Kostheim (DE)
(74) Vertreter: Klunker . Schmitt-Nilson . Hirsch

(56) Entgegenhaltungen:
- EP-A- 0 593 212
- DE-C- 19 616 358
- FR-A- 1 316 616
- FR-A- 1 468 833
- US-A1- 2002 037 240

## Beschreibung

Die Erfindung betrifft ein Warenpräsentationsmöbel, aufweisend wenigstens einen gekühlten Warenraum und wenigstens eine Vorrichtung zur Erzeugung eines in dem Warenpräsentationsmöbel zirkulierenden Kühlluftstromes, der der Kühlung des oder der Warenräume dient, wobei der oder den Vorrichtungen zur Erzeugung eines in dem Warenpräsentationsmöbel zirkulierenden Kühlluftstromes wenigstens ein Ozongenerator derart zugeordnet ist, dass das durch den oder die Ozongeneratoren gebildete Ozon dem oder den zirkulierenden Kühlluftströmen beigemischt wird.

Ferner betrifft die Erfindung ein Verfahren zum Betreiben eines Warenpräsentationsmöbels.

Unter dem Begriff "Vorrichtung zur Erzeugung eines in dem Warenpräsentationsmöbel zirkulierenden Kühlluftstromes" seien diejenigen Bauteile, wie Verdampfer, Verdichter, Ventilator, etc. zu verstehen, die innerhalb des Warenpräsentationsmöbels zum Zwecke der Erzeugung des zirkulierenden Kühlluftstromes vorgesehen sind. Abhängig davon, ob es sich bei dem jeweiligen Warenpräsentationsmöbel um ein so genanntes steckerfertiges oder ein so genanntes nicht steckerfertiges Möbel handelt, können bestimmte der vorgenannten Bauteile auch entfallen.

Allen bekannten Warenpräsentationsmöbeln ist gemein, dass sich in den Warenräumen, denjenigen Bereichen, die von den zirkulierenden Kühlluftströmen überstrichen werden, sowie auf Bauteilen, wie Verdampfer, etc., Mikroorganismen und Keime bilden und anlagern.

Dies führt dazu, dass die Haltbarkeit bestimmter Lebensmittel verringert wird. Femer können derartige Mikroorganismen bzw. Keime auch zu einer für den Kunden sowie das Bedienpersonal schädlichen, gesundheitlichen Belastungen - dies gilt insbesondere für Allergiker und Asthmatiker - sowie unangenehmen Geruchsbelästigung führen.

Bisher werden Warenpräsentationsmöbel deshalb in mehr oder weniger regelmäßigen Abständen händisch gereinigt bzw. desinfiziert. Allerdings lassen sich einige Bauteile, wie beispielsweise Verdampfer, und Bereiche nur mit einem unverhältnismäßig großem Aufwand reinigen, da dazu zunächst Abdeckungen entfernt werden müssten. Aber auch die bei der Reinigung bzw. Desinfizierung verwendeten Reinigungs- bzw. Desinfektionsmittel sind nicht vollständig frei von Komponenten, deren Kontakt mit den präsentierten Waren vermieden werden sollten.

Darüber hinaus ist zu bedenken, dass das händische Reinigen bzw. Desinfizieren von Warenpräsentationsmöbeln zum einen vergleichsweise zeitaufwendig ist und zum anderen nicht sichergestellt werden kann, dass auch wirklich alle Bereiche, bei denen dies erforderlich ist, ausreichend gereinigt bzw. desinfiziert worden sind. US 2002/00 37 240 A1 offenbart eine Kühleinrichtung gemäß dem Oberbegriff von Anspruch 1.

Aufgabe der vorliegenden Erfindung ist es, ein gattungsgemäßes Warenpräsentationsmöbel sowie ein gattungsgemäßes Verfahren zum Betreiben eines Warenpräsentationsmöbels anzugeben, das die vorgenannten Probleme vermeidet, insbesondere das Problem der nur ungenügenden Reinigung schwer zugänglicher Bauteile und Bereiche löst.

Zur Lösung dieser Aufgabe wird ein gattungsgemäßes Warenpräsentationsmöbel vorgeschlagen, das Mittel zur Erfassung des Verschmutzungsgrades des- oder derjenigen Bereiche, die mit der zu kühlenden Ware in Kontakt kommen, aufweist, wobei die Mittel zur Erfassung des Verschmutzungsgrades mit dem oder den Ozongeneratoren in Wirkverbindung stehen.

Das erfindungsgemäße Verfahren zum Betreiben eines Warenpräsentationsmöbels ist dadurch gekennzeichnet, dass in Abhängigkeit von dem erfassten Verschmutzungsgrad die Leistung des oder der Ozongeneratoren variiert wird.

Ozon ist nach Fluor das stärkste, bekannte Oxidationsmittel und vermag sowohl Keime abzutöten als auch Geruchsstoffe zu oxidieren und somit zu neutralisieren.

Ozongeneratoren unterschiedlichster Bauart sind dem Fachmann aus dem Stand der Technik bekannt. Ozongeneratoren neuerer Bauart besitzen eine hohe Lebensdauer und bedürfen darüber hinaus eines nur geringen Wartungsaufwandes.

Innerhalb des Warenpräsentationsmöbels wird wenigstens ein Ozongenerator der oder den Vorrichtungen zur Erzeugung des in dem Warenpräsentationsmöbel zirkulierenden Kühlluftstromes derart zugeordnet, dass das durch ihn gebildete Ozon dem oder den zirkulierenden Kühlluftströmen beigemischt wird. In Abhängigkeit von der Konstruktion des Warenpräsentationsmöbels - insbesondere von dessen Breite - sind unter Umständen mehrere Ozongeneratoren vorzusehen, da mittels lediglich eines Ozongenerators keine gleichmäßig Verteilung des gebildeten Ozons in dem oder den zirkulierenden Kühlluftströmen erreicht werden kann.

Das gebildete und dem oder den zirkulierenden Kühlluftströmen beigemischte Ozon bildet zusammen mit dem oder den zirkulierenden Kühlluftströmen eine "Schutzgasatmosphäre", die die Bildung bzw. Anlagerung von Keimen und Mikroorganismen wirkungsvoll verhindert.

Das mittels des oder der Ozongeneratoren gebildete Ozon wird nunmehr durch den oder die zirkulierenden Kühlluftströme aufgenommen und über diejenigen Flächen und Bereiche des Warenpräsentationsmöbels geführt, die auch mit dem Kühlluftstrom in Kontakt kommen. Die vorgenannten Flächen und Bereiche werden auf diese Weise durch das im Kreislauf geführte Ozon "gereinigt".

Das erfindungsgemäße Warenpräsentationsmöbel weiterbildend wird vorgeschlagen, dass der oder die Ozongeneratoren hinsichtlich ihrer Leistungen variierbar ausgebildet sind.

Insbesondere in der Zusammenschau der vorgenannten Ausgestaltungen des erfindungsgemäßen Warenpräsentationsmöbels lässt sich der Ozonisierungsgrad in dem oder den zirkulierenden Kühlluftströmen in Abhängigkeit von dem erfassten Verschmutzungsgrad beeinflussen und variieren. Somit kann die Leistung des oder der Ozongeneratoren dem erfassten Verschmutzungsgrad angepasst werden, was zur Folge hat, dass immer nur die für eine ausreichende Reinigung bzw. Desinfizierung erforderliche Ozonmenge gebildet wird.

Entsprechend einer vorteilhaften Ausgestaltung des erfindungsgemäßen Verfahrens zum Betreiben eines Warenpräsentationsmöbels, wird in regelmäßigen oder unregelmäßigen Abständen die Leistung des oder zumindest eines der Ozongeneratoren erhöht.

Mittels dieser vorteilhaften Ausgestaltung des erfindungsgemäßen Verfahrens zum Betreiben eines Warenpräsentationsmöbels kann in (vor)einstellbaren oder beliebigen zeitlichen Abständen eine intensivere Reinigung des Warenpräsentationsmöbels realisiert werden.

Im Regelfall wird durch den Einsatz eines Ozongenerators täglich im Wesentlichen ununterbrochen eine schützende Gasatmosphäre, die die Bildung von unerwünschten Keimen und Bakterien verhindert, erzeugt. Zusätzlich wird vorzugsweise wenigstens einmal pro Tag eine intensive Reinigung des Warenpräsentationsmöbels realisiert, indem die Leistung des oder zumindest eines der Ozongeneratoren erhöht wird.

In vorteilhafter Weise erfolgt die vorbeschriebene Intensivreinigung in Kombination mit der Abtauphase des oder eines der Verdampfer des erfindungsgemäßen Warenpräsentationsmöbels. Dadurch wird eine Steigerung der Effektivität der Intensivreinigung erreicht, da es zu einer Sekundärreaktion kommt.

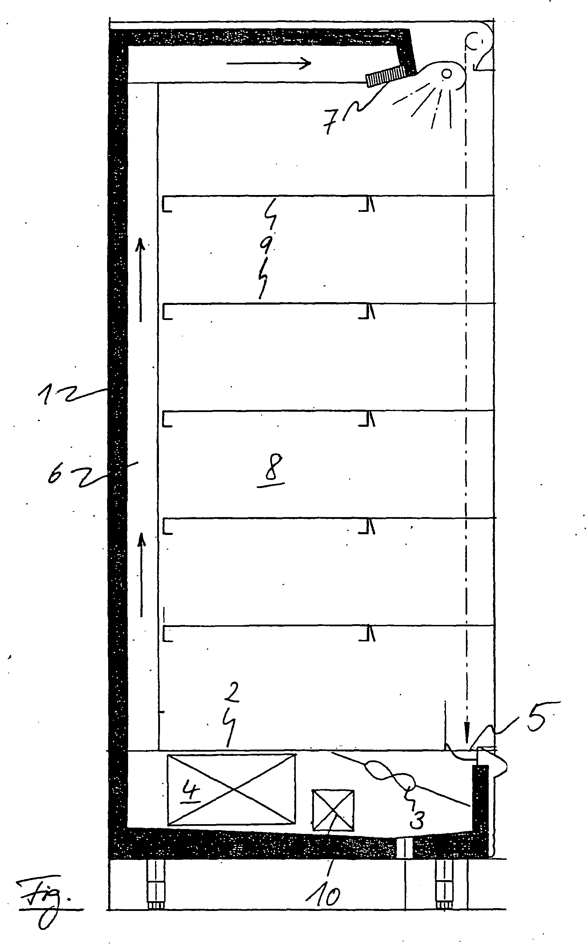

Das erfindungsgemäße Warenpräsentationsmöbel, das erfindungsgemäße Verfahren zum Betreiben eines Warenpräsentationsmöbel sowie weitere Ausgestaltungen desselben seien nachfolgend anhand des in der Figur dargestellten Ausführungsbeispieles näher erläutert. Hierbei zeigt die Figur eine schematisierte, seitliche Schnittdarstellung durch eine mögliche Ausführungsform eines Kühlregales.

Kühlregale weisen einen isolierten Grundkörper 1, der den gekühlten Warenraum 8 umgibt, auf. Innerhalb des gekühlten Warenraumes 8 sind mehrere Warenpräsentationsböden 9 angeordnet.

Im Regelfall unterhalb des untersten Warenpräsentationsbodens 2 sind die für die Erzeugung des bzw. der Kühlluftströme erforderlichen Aggregate angeordnet; in der Figur sind der Übersichtlichkeit halber lediglich ein Ventilator 3 sowie ein Verdampfer bzw. Wärmetauscher 4 dargestellt.

Der über eine im vorderen, unteren Bereich des Kühlregales angeordnete Öffnung 5 eintretende Kühlluftschleier wird mittels des Ventilators 3 durch den Verdampfer bzw. Wärmetauscher 4 geführt und in diesem abgekühlt. Anschließend wird der Kühlluftstrom über den Zuluftkanal 6 zu der im vorderen, oberen Bereich des Kühlregales angeordneten Luftaustrittswabe 7, über die er austritt und entlang der Warenraumöffnung streicht, geführt.

Weiterhin ist wenigstens ein Ozongenerator 10 vorgesehen. Dieser ist - wie in der Figur dargestellt - vorzugsweise im Bereich des Ventilators 3 oder an einem anderen strömungsoptimierten Ort angeordnet, so dass er in bestmöglicher Weise von der in dem Kühlregal zirkulierenden Kühlluft umspült wird bzw. das von ihm gebildete Ozon mit der zirkulierenden Kühlluft in Kontakt kommt. Durch eine derartige Anordnung lässt sich eine gleichmäßige Verteilung des mittels des Ozongenerators 10 gebildeten Ozons in dem zirkulierenden Kühlluftstrom erreichen.

In vorteilhafter Weise sind - wie in der Figur dargestellt - der oder die Ozongeneratoren 10 bei Warenpräsentationsmöbeln, die wenigstens einen Verdampfer oder Wärmetauscher 4 aufweisen, in Strömungsrichtung vor dem Verdampfer oder Wärmetauscher 4 angeordnet.

Diese Anordnung - die eine besonders intensive Reinigung der Verdampfer bzw. Wärmetauscher ermöglicht - ist deshalb von großem Vorteil, da Verdampfer bzw. Wärmetauscher, diejenigen Bauteile bzw. Komponenten innerhalb eines Warenpräsentationsmöbels darstellen, die am stärksten verschmutzen, aber für das Reinigungspersonal praktisch nicht zugänglich sind.

Prinzipiell kann bzw. können die vorgesehenen Ozongeneratoren jedoch auch an anderen geeigneten Stellen innerhalb des Warenpräsentationsmöbels angeordnet werden. Diese Anordnung des bzw. der Ozongeneratoren wird in der Praxis von der Art des jeweiligen Warenpräsentationsmöbels abhängen.

Allgemein gilt, dass - entsprechend einer vorteilhaften Ausgestaltung des erfindungsgemäßen Warenpräsentationsmöbels - der oder die Ozongeneratoren 10 in dem bzw. denjenigen Bereichen, die von dem oder den zirkulierenden Kühlluftströmen am stärksten durch- oder überströmt werden, angeordnet sein sollten. Dadurch wird eine bestmögliche Verteilung des erzeugten Ozons in dem oder den zirkulierenden Kühlluftströmen erreicht, woraus eine optimale Reinigungswirkung resultiert.

Das erfindungsgemäße Warenpräsentationsmöbel weiterbildend wird vorgeschlagen, das Warenpräsentationsmöbel Mittel zur Überwachung des oder der Ozongeneratoren 10 und/oder Mittel zur Überwachung der Mittel zur Erfassung des Verschmutzungsgrades aufweist.

In analoger Weise wird das erfindungsgemäße Verfahren zum Betreiben des erfindungsgemäßen Warenpräsentationsmöbels, wobei das Warenpräsentationsmöbel Mittel zur Überwachung des oder der Ozongeneratoren (10) und/oder Mittel zur Überwachung der Mittel zur Erfassung des Verschmutzungsgrades aufweist, weiterbildend vorgeschlagen, dass die Mittel zur Überwachung in eine lokale und/oder Femüberwachung des Warenpräsentationsmöbels integriert sind.

Die vorgenannten Ausgestaltungen des erfindungsgemäßen Warenpräsentationsmöbels bzw. des erfindungsgemäßen Verfahrens ermöglichen es nunmehr, den hygienischen Zustand des Warenpräsentationsmöbels bzw. seines Warenraumes lokal und/oder mittels Datenfernübertragung permanent oder in (regelmäßigen) Abständen zu überwachen, entsprechende Nachregelungen vorzunehmen und den jeweiligen Zustand zu dokumentieren. Die hierfür innerhalb des Warenpräsentationsmöbels erforderliche Elektronik und ggf. auch Sensorik kann in der im Regelfall bereits vorhandenen Elektronik bzw. Sensorik integriert werden.

Werden innerhalb eines Supermarktes eine Vielzahl von Warenpräsentationsmöbeln vorgesehen, so wird oftmals ein so genannter Zentralrechner vorgesehen, an den die Regelelektroniken der einzelnen Warenpräsentationsmöbel und damit auch die Mittel zur Überwachung bzw. deren Elektronik angebunden sind.

Mittels der Erfindung wird somit ein Warenpräsentationsmöbel geschaffen, das sich - zumindest in denjenigen Bereichen, die von dem oder den zirkulierenden Kühlluftströmen überstrichen werden - selbst reinigt. Auf den gereinigten Flächen bzw. in den gereinigten Bereichen verbleiben dabei keine Rückstände von Reinigungs- oder Desinfektionsmitteln. Auch wird die Bildung von möglicherweise unangenehmen Gerüchen durch die Erfindung wirkungsvoll verhindert; femer werden vorhandene (unangenehme) Gerüche neutralisiert. Darüber hinaus kann bei einer Vielzahl von Lebensmitteln deren Haltbarkeit verlängert werden.

Des Weiteren wird zudem der Keimgehalt in dem Ladengeschäft bzw. Supermarkt, in dem das erfindungsgemäße Warenpräsentationsmöbel steht, ebenfalls positiv beeinflusst, da zwischen dem zirkulierenden Kühlluftstrom und der das erfindungsgemäße Warenpräsentationsmöbel umgebenden Raumluft ein permanenter Luftaustausch stattfindet.

Es sei betont, dass sich die Erfindung sowohl bei den so genannten steckerfertigen als auch den so genannten nicht steckerfertigen Warenpräsentationsmöbeln realisieren lässt.

## Patentansprüche

1. Warenpräsentationsmöbel, aufweisend wenigstens einen gekühlten Warenraum und wenigstens eine Vorrichtung zur Erzeugung eines in dem Warenpräsentationsmöbel zirkulierenden Kühlluftstromes, der der Kühlung des oder der Warenräume dient, wobei der oder den Vorrichtungen zur Erzeugung (3, 4) eines in dem Warenpräsentationsmöbel zirkulierenden Kühlluftstromes wenigstens ein Ozongenerator (10) derart zugeordnet ist, dass das durch den oder die Ozongeneratoren (10) gebildete Ozon dem oder den zirkulierenden Kühlluftströmen beigemischt wird, **dadurch gekennzeichnet, dass** das Warenpräsentationsmöbel Mittel zur Erfassung des Verschmutzungsgrades des- oder derjenigen Bereiche, die mit der zu kühlenden Ware in Kontakt kommen, aufweist und die Mittel zur Erfassung des Verschmutzungsgrades mit dem oder den Ozongeneratoren (10) in Wirkverbindung stehen.

2. Warenpräsentationsmöbel nach Anspruch 1, **dadurch gekennzeichnet, dass** der oder die Ozongeneratoren (10) hinsichtlich ihrer Leistungen variierbar ausgebildet sind.
Warenpräsentationsmöbel nach Anspruch 1 oder 2.

3. Warenpräsentationsmöbel nach einem der vorhergehenden Ansprüche 1 bis 2, **dadurch gekennzeichnet, dass** der oder die Ozongeneratoren (10) in unmittelbarer Nähe zum dem oder einem der Ventilatoren (3) angeordnet sind.

4. Warenpräsentationsmöbel nach einem der vorhergehenden Ansprüche 1 bis 3, wobei das Warenpräsentationsmöbel wenigstens einen Verdampfer oder Wärmetauscher aufweist, **dadurch gekennzeichnet, dass** der oder die Ozongeneratoren (10) in Strömungsrichtung vor dem Verdampfer oder Wärmetauscher (4) angeordnet sind.

5. Warenpräsentationsmöbel nach einem der vorhergehenden Ansprüche 1 bis 4 **dadurch gekennzeichnet, dass** der oder die Ozongeneratoren (10) in dem bzw. denjenigen Bereichen, die von dem oder den zirkulierenden Kühlluftströmen am stärksten durch- oder überströmt werden, angeordnet sind.

6. Warenpräsentationsmöbel nach einem der vorhergehenden Ansprüche 1 bis 5, **dadurch gekennzeichnet, dass** das Warenpräsentationsmöbel Mittel zur Überwachung des oder der Ozongeneratoren (10) und/oder Mittel zur Überwachung der Mittel zur Erfassung des Verschmutzungsgrades aufweist.

7. Verfahren zum Betreiben eines Warenpräsentationsmöbels nach einem der vorhergehenden Ansprüche 1 bis 6, **dadurch gekennzeichnet, dass** in Abhängigkeit von dem erfassten Verschmutzungsgrad die Leistung des oder der Ozongeneratoren (10) variiert wird.

8. Verfahren nach Anspruch 7, **dadurch gekennzeichnet, dass** in regelmäßigen oder unregelmäßigen Abständen die Leistung des oder zumindest eines der Ozongeneratoren (10) erhöht wird.

9. Verfahren nach Anspruch 8, wobei der oder zumindest einer der Verdampfer oder Wärmetauscher des Warenpräsentationsmöbels in regelmäßigen oder unregelmäßigen Abständen einem Abtauprozess unterworfen wird, **dadurch gekennzeichnet, dass** die in regelmäßigen oder unregelmäßigen Abständen erfolgende Erhöhung der Leistung des oder zumindest eines der Ozongeneratoren (10) im Wesentlichen zeitgleich mit dem oder einem Abtauprozess erfolgt.

10. Verfahren nach einem der vorhergehenden Ansprüche 7 bis 9, wobei das Warenpräsentationsmöbel Mittel zur Überwachung des oder der Ozongeneratoren (10) und/oder Mittel zur Überwachung der Mittel zur Erfassung des Verschmutzungsgrades aufweist, **dadurch gekennzeichnet, dass** die Mittel zur Überwachung in eine lokale und/oder Femüberwachung des Warenpräsentationsmöbels integriert sind.

## Claims

1. Goods show case, comprising at least one refrigerated goods space and at least one device for generating a current of chilled air circulating in the goods show case, which serves to chill the goods space or spaces, wherein at least one ozone generator (10) is associated with the device or devices (3, 4) for generating a current of chilled air circulating in the goods show case such that the ozone formed by the ozone generator or generators (10) is mixed with the circulating current or currents of chilled air, **characterised in that** the goods show case comprises means for detecting the degree of soiling of the area or areas which come into contact with the goods to be refrigerated, and the means for detecting the degree of soiling are operatively connected to the ozone generator or generators (10).

2. Goods show case according to claim 1, **characterised in that** the ozone generator or generators (10) are constructed to be variable in their power output.

3. Goods show case according to one of the preceding claims 1 to 2, **characterised in that** the ozone generator or generators (10) are arranged in the immediate vicinity of the ventilator (3) or one of the ventilators (3).

4. Goods show case according to one of the preceding claims 1 to 3, wherein the goods show case comprises at least one evaporator or heat exchanger, **characterised in that** the ozone generator or generators (10) are arranged in front of the evaporator or heat exchanger (4) in the direction of flow.

5. Goods show case according to one of the preceding claims 1 to 4, **characterised in that** the ozone generator or generators (10) are arranged in the area or areas through which or over which the circulating current or currents of chilled air flow most strongly.

6. Goods show case according to one of the preceding claims 1 to 5, **characterised in that** the goods show case comprises means for monitoring the ozone generator or generators (10) and/or means for monitoring the means for detecting the degree of soiling.

7. Method of operating goods show case according to one of the preceding claims 1 to 6, **characterised in that** the power output of the ozone generator or generators (10) is varied as a function of the degree of soiling detected.

8. Method according to claim 7, **characterised in that** the power output of the ozone generator or at least one of the ozone generators (10) is increased at regular or irregular intervals.

9. Method according to claim 8, wherein the evaporator or heat exchanger or at least one of the evaporators or heat exchangers of the goods show case is subjected to a defrosting process at regular or irregular intervals, **characterised in that** the increase in the power of the ozone generator or at least one of the ozone generators (10) occurs at substantially the same time as the or a defrosting process.

10. Method according to one of the preceding claims 7 to 9, wherein the goods show case comprises means for monitoring the ozone generator or generators (10) and/or means for monitoring the means for detecting the degree of soiling, **characterised in that** the monitoring means are integrated in a local and/or remote monitoring of the goods show case.

## Revendications

1. Meuble d'étalage de marchandises comportant au moins un compartiment de marchandises réfrigéré et au moins un dispositif destiné à générer un flux d'air froid circulant dans le meuble d'étalage de marchandises, lequel a pour fonction de refroidir le ou les compartiments de marchandises, au moins un générateur d'ozone (10) étant associé au ou aux dispositifs (3, 4) destinés à générer un flux d'air froid circulant dans le meuble d'étalage de marchandises, de telle sorte que l'ozone produit par le ou les générateurs d'ozone (10) est mélangé au ou aux flux d'air froid en circulation, **caractérisé en ce que** le meuble d'étalage de marchandises comporte des moyens destinés à détecter le degré de pollution de la ou des zones qui viennent en contact avec la marchandise à réfrigérer et les moyens destinés à détecter le degré de pollution sont en liaison active avec le ou les générateurs d'ozone (10).

2. Meuble d'étalage de marchandises selon la revendication 1, **caractérisé en ce que** le ou les générateurs d'ozone (10) sont réalisés de manière à pouvoir varier leur puissance.

3. Meuble d'étalage de marchandises selon l'une quelconque des revendications 1 à 2, **caractérisé en ce que** le ou les générateurs d'ozone (10) sont disposés à proximité immédiate du ou d'un des ventilateurs (3).

4. Meuble d'étalage de marchandises selon l'une quelconque des revendications 1 à 3, le meuble d'étalage de marchandises comportant au moins un évaporateur ou échangeur thermique, **caractérisé en ce que** le ou les générateurs d'ozone (10) sont disposés par référence à la direction d'écoulement en amont de l'évaporateur ou échangeur thermique (4).

5. Meuble d'étalage de marchandises selon l'une quelconque des revendications 1 à 4, **caractérisé en ce que** le ou les générateurs d'ozone (10) sont montés dans la ou les zones qui sont le plus exposées à la circulation ou au balayage du ou des flux d'air froid.

6. Meuble d'étalage de marchandises selon l'une quelconque des revendications 1 à 5, **caractérisé en ce que** le meuble d'étalage de marchandises comporte des moyens destinés à contrôler le ou les générateurs d'ozone (10) et/ou des moyens destinés à contrôler les moyens de détection du degré de pollution.

7. Procédé d'exploitation d'un meuble d'étalage de marchandises selon l'une quelconque des revendications 1 à 6, **caractérisé en ce que** la puissance du ou des générateurs d'ozone (10) varie en fonction du degré de pollution détecté.

8. Procédé selon la revendication 7, **caractérisé en ce que** la puissance du ou des générateurs d'ozone (10) est augmentée à intervalles réguliers ou irréguliers.

9. Procédé selon la revendication 8, le ou au moins un évaporateur ou échangeur thermique du meuble d'étalage de marchandises étant soumis à intervalles réguliers ou irréguliers à un processus de dégivrage, **caractérisé en ce que** l'augmentation de la puissance du ou des générateurs d'ozone (10), effectuée à intervalles réguliers ou irréguliers, est effectuée sensiblement en même temps que le ou un processus de dégivrage.

10. Procédé selon l'une quelconque des revendications 7 à 9, le meuble d'étalage de marchandises comportant des moyens destinés à contrôler le ou les générateurs d'ozone (10) et/ou des moyens destinés à contrôler les moyens de détection du degré de pollution, **caractérisé en ce que** les moyens de contrôle sont intégrés dans une surveillance locale et/ou télésurveillance du meuble d'étalage de marchandises.
